# EUROPEAN PATENT SPECIFICATION

(11) **EP 0 746 340 B1**
(45) Date of publication and mention of the grant of the patent: **07.11.2007**
(21) Application number: 95909606.6
(22) Date of filing: 24.02.1995
(51) Int. Cl.: A61K 51/08

(54) **PEPTIDE-CHELATOR CONJUGATES**
PEPTID-CHELATOR KONJUGATE
PRODUITS DE CONJUGAISON PEPTIDES-CHELATES

(30) Priority: 25.02.1994 US 202178
(43) Date of publication of application: 11.12.1996
(73) Proprietor: BRACCO International B.V., 1077 ZX Amsterdam (NL)
(72) Inventor: GOODBODY, Anne, Toronto, Ontario M4L 2X7 (CA); POLLAK, Alfred, Toronto, Ontario M6B 4C6 (CA)
(74) Representative: Bassil, Nicholas Charles
(86) International application number: PCT/CA1995/000106
(87) International publication number: WO 1995/022996

(56) References cited:
- WO-A-93/25244
- WO-A-95/03280
- WO-A-95/13832
- WO-A-95/17419
- LYMPHOKINE REP, 1980, VOL. 1, PAGE(S) 157-179, NAJJAR VA ET AL 'THE CHEMISTRY AND BIOLOGY OF TUFTSIN'
- PROC. NATL ACAD. SCI. U. S. A., 1986, VOL. 83, NO. 19, PAGE(S) 7187-7191, BUMP N.J. ET AL 'Isolation and subunit composition of tuftsin receptor'
- DATABASE CANCERLIT Host: DIALOG AN = 384993, KONOPINSKA D ET AL 'ELONGATED TUFTSIN ANALOGUES --SYNTHESIS AND BIOLOGICAL INVESTIGATION' & ANN N Y ACAD SCI, 1983, VOL. 419, PAGE(S) 35-43,
- DATABASE EMBASE ELSEVIER SCIENCE PUBLISHERS, AMSTERDAM, NL AN = 5599439, FLORENTIN I. ET AL 'Immunopharmacological properties of tuftsin and of some analogues' & ANN. N.Y. ACAD. SCI., 1983, VOL. 419, PAGE(S) 177-191,

## Description

### FIELD OF THE INVENTION

This invention is in the field of diagnostic imaging, and relates to a peptide targetting agent useful for targetting sites of inflammation.

### BACKGROUND OF THE INVENTION

The art of diagnostic imaging exploits targetting agents that in binding or localizing sites selectively within the body, help to resolve the image of diagnostic interest. Monoclonal antibodies for example have been developed to have high affinity and specificity for particular cancer cells and therefore are useful for imaging tumours. Despite high affinity and specificity, antibodies do not provide ideal imaging agents since they are costly to produce on a commercial scale as well as their poor labelling characteristics. In particular, metal labels tend to bind at numerous low-affinity binding sites on antibodies and are released in vivo resulting in undesirable accumulation of the label at non-target sites. An alternative targetting agent to antibodies are small receptor binding peptides. Peptides offer the advantage of efficient labelling facilitated by conjugation to various chelating molecules. Other advantages of peptides over antibodies is their ease of synthesis, rapid tissue penetration and rapid clearance from the body.

A naturally occurring tetrapeptide, tuftsin TKPR, was discovered to stimulate phagocytosis by binding to receptors expressed on the outer surface of neutrophils and macrophages. Phagocytosis constitutes a major line of defense for a host against bacterial infections, therefore as a stimulator of phagocytosis tuftsin would be expected to be a good peptide for imaging sites of infectious inflammation. However, studies show that tuftsin labelled with a radionuclide metal undesirably accumulates in non-target tissue. In particular, labelled tuftsin accumulates in the gastrointestinal tract which limits its usefulness as an imaging agent.

In light of the difficulties associated with antibodies, it would be desirable to provide a peptidic targetting agent capable of localizing at sites of inflammation while not having substantial accumulation in non-target tissue.

Najjar et al, "The Chemistry and Biology of Tuftsin" Lymphokine Rep, 1980, Vol 1, pp 157-179 generally discusses the chemistry and biology of tuftsin, a cytoactive tetrapeptide found in many living organisms. Najjar *et al* also discusses in detail the properties of tuftsin and mentions the tuftsin analog, TKPPR.

Bump et al, "Isolation and subunit composition of tuftsin receptor" Proc. Natl. Acad. Sci. USA, 1986, Vol 83, No. 19, pp 7187-7191 relates to the purification of the tuftsin receptor using the analog TKPPR bound to a solid support. Bump *et al* found that TKPPR binds tuftsin receptor with 4 times the avidity *in vitro* than tuftsin.

WO A 93/15771 relates to amide-thiolate ligands having improved metal chelate formation kinetics. These ligands were suggested to be useful for post formed labeling of biological substances for use in diagnosis and therapy.

### SUMMARY OF THE INVENTION

Peptide-chelator conjugates are provided that when labelled with a traceable metal are useful for diagnostic imaging of sites of inflammation. The peptide component is an antagonist of the naturally occurring tetrapeptide tuftsin while the chelator component serves as a labelling site for metals, in particular radionuclide metals such as technetium-99m. According to an aspect of the invention, there are provided peptide-chelator conjugates in which Thr-X-Pro-Pro-Arg is coupled to a metal chelator, wherein X is an amino acid residue or an analogue thereof, provided that the peptide chelator conjugate is not:
Pic-Ser-Cys-Gly-TKPPR;
Pic-Ser-Cys(Acm)- Gly)-TKPPR;
2-Quinolinic acid-Ser-Cys(Acm)-Gly-TKPPR;
1-Quinolinic acid-Ser-Cys(Acm)-Gly-TKPPR;
3-Quinolinic acid-Ser-Cys(Acm)-Gly-TKPPR;
Indole-2-carboxylic acid-Ser-Cys(Acm)-Gly-TKPPR;
Pyrrole-2-carboxylic acid-Ser-Cys(Acm)-Gly-TKPPR;
Ser-Cys-Gly-TKPPR; or
S-Acm-Mercaptoacetyl-Ser-N-methylhydrazino-nicotinic acid-Gly-Thr-Lys-Pro-Pro-Arg

In a particular embodiment of the present invention, the metal chelator component of the conjugate is of the formula 1: 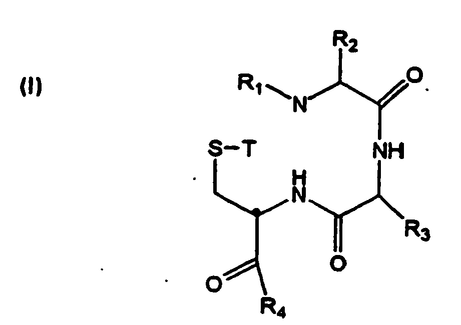 wherein
R₁ and R₂ together form a 5- or 6-membered heterocyclic ring which is optionally fused to a 5- or 6-membered ring wherein either ring is optionally substituted with groups selected from alkyl, alkoxy, carboxyl, halogen, hydroxyl and a linking group;
R₃ is selected from H; alkyl; and alkyl substituted by a group selected from amino, aminoacyl, carboxyl, guanidinyl, hydroxyl, thiol, phenyl, phenolyl, indolyl and imidazolyl;
R₄ is selected from hydroxyl, alkoxy, and a linking group; and
T represents H or a sulfur protecting group.

In a particular embodiment of the present invention, the metal chelator component of the conjugate is of the formula II: 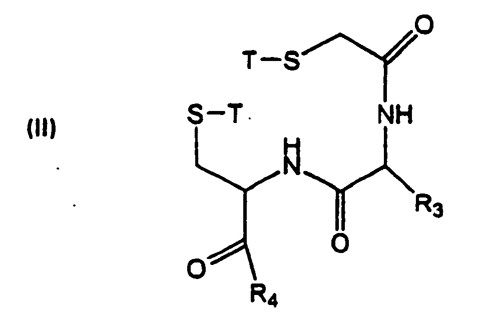 wherein
R₃, R₄ and T are as defined above; and

According to an aspect of the invention, the peptide-chelator conjugates are provided in a form complexed with a diagnostically useful metal or an oxide or nitride thereof.

According to another aspect of the present invention, there is provided the use of a diagnostically effective amount of a composition comprising a peptide-chelator conjugate as described above in the preparation of an agent for imaging a site of inflammation in a mammal.

### DETAILED DESCRIPTION OF THE INVENTION

The invention provides peptide-chelator conjugates that when complexed with a diagnostically useful metal are useful for imaging sites of inflammation. The peptide-chelator conjugate, also referred to as a "conjugate", incorporates as the peptide component, an antagonist of tuftsin coupled to a metal chelator, the peptide component consisting of the amino acid sequence Thr-X-Pro-Pro-Arg (TXPPR) wherein X is a naturally occurring or non-naturally occurring amino acid residue. In a particular embodiment, X is an amino acid residue having an α-carbon side chain that is charged or polar under physiological conditions. Preferably, X is selected from the group of amino acid residues lysine (Lys or K), glutamine (Gin or Q), arginine (Arg or R), asparagine (Asn or N), glutamate (Glu or E), aspartate (Asp or D), tyrosine (Tyr or Y) and threonine (Thr or T). More preferably, X is selected from lysine, glutamine, arginine and asparagine. Most preferably, X is selected from lysine and glutamine.

Amino acid residues that are non-naturally occurring are also encompassed by X. Suitable non-naturally occurring residues are those that can replace lysine or glutamine without disrupting biodistribution properties of the conjugates. It will be appreciated from the fact that replacement of lysine with glutamine is well tolerated for imaging purposes, that X can vary widely in terms of side chain structure. In particular, non-naturally occurring amino acid residues include those that differ from natural residues in the length of their side chain including lysine, glutamine, arginine, asparagine, glutamate, aspartate, tyrosine and threonine. The side chain of a non-naturally occurring residue will incorporate a C₁₋₈alkylene chain that may be branched with one or two C₁₋₂alkyl groups. Preferably the alkylene chain will have 1-8 methylene groups and will differ from a corresponding natural residue by 1 or 2 methylene groups. Preferably, non-naturally occurring residues include lysine and glutamine having one additional or fewer methylene groups in their side chain. Suitable non-naturally occurring residues are commercially available or can be synthesized according to established chemical techniques.

It is understood that the chelator may be coupled to either the N- or C-terminums of the peptide Thr-X-Pro-Pro-Arg. When coupled to the chelator at its N-terminus, the peptide Thr-X-Pro-Pro-Arg may be extended at its C-terminus preferably by 1 to 3 amino acid residues or may be modified at the C-terminus, for example amidated or otherwise derivatized to prevent digestion by exopeptidase. Acceptable extensions or modifications are those which do not detract appreciably from the ability of the conjugate to image inflammation, as determined by the assessment model herein described.

For diagnostic imaging purposes, a chelator is a compound that binds to a radionuclide metal to form a complex that is stable under physiological conditions and also has a reactive functional group for conjugation with a targeting molecule. Chelators of the radionuclide metal ^{99m}Tc, typically incorporate a combination of four nitrogen and sulfur metal-coordinating atoms and are designated as N₄, N₃S, N₂S₂ etc. However, chelators may incorporate other metal-coordinating atoms such as oxygen, phosphorous and selenium. For convenient synthesis of conjugates of the invention, chelators are ideally peptide-based, so that the conjugates can be synthesized *in toto* using solid-phase peptide synthesis techniques.

In an embodiment of the present invention, the peptide is coupled at its N-terminus to the N₃S-type metal chelators of formula (I) illustrated above, the manufacture and use of which are disclosed in co-pending United States application in the name of Pollak et al, filed on 22 December 1993, Serial Number 08,171,737. In another embodiment, the peptide is coupled at its N-terminus to N₂S₂-type metal chelators of formula (II). The terms defining the variables R₁ - R₄ and T as used hereinabove in formula (I) and (II) have the following meanings:
"alkyl" refers to a straight or branched C₁-C₈ chain and includes lower C₁-C₄ alkyl;
"alkoxy" refers to straight or branched C₁-C₈ alkoxy and includes lower C₁-C₄ alkoxy;
"thiol" refers to a sulfhydryl group that may be substituted with an alkyl group to form a thioether;
"sulfur protecting group" refers to a chemical group that is bonded to a sulfur atom and inhibits oxidation of sulfur and includes groups that are cleaved upon chelation of the metal. Suitable sulfur protecting groups include known alkyl, aryl, acyl, alkanoyl, aryloyl, mercaptoacyl and organothio groups.
"linking group" refers to a chemical group that serves to couple the peptide to the chelator while not adversely affecting either the targetting function of the peptide or the metal binding function of the chelator. Suitable linking groups include alkyl chains and amino acid chains functionalized with a reactive groups for coupling to the peptide or chelator.
"metal chelator" refers to a molecule that forms a stable complex with a traceable metal atom under physiological conditions in that the metal remains bound to the conjugate in vivo.

In preferred embodiments of the invention, the chelators conform to the above formulae (I) or (II) in which: R₁ and R₂ together form a six-membered heterocyclic ring; R₃ is selected from H and a hydroxyl substituted alkyl group selected from methyl and ethyl and most preferably hydroxymethyl; R₄ is a linking group of one to three amino acid residues and T is the sulfur protecting group acetamidomethyl (Acm) or benzoyl (Bz);

In more preferred embodiments of the invention, the chelators conform to the above formula (I) wherein R₁ and R₂ together form a pyridine ring; R₃ is hydroxymethyl; T is Acm and R₄ is a linking group selected from -Gly- and - Gly-Asp-Gly-. These chelators in a form coupled to the peptide are represented by the sequences:
Pic-Ser-Cys (Acm)-Gly- TKPPR;
Pic-Ser-Cys(Acm)-Gly-TQPPR; and
Pic-Ser-Cys(Acm)-Gly-Asp-Gly-TKPPR;
wherein Pic represents the amino acid derivative picolinic acid.

In a preferred embodiment of the invention, the chelators conform to the above formula (II) wherein R₃ is hydroxymethyl; T is Acm or Bz and R₄ is the linking group -Gly-Asp-Gly-. This chelator coupled to the peptide at the linking group of R₄ is represented by the sequence:
Bz-MA-Ser-Cys-Ser-Gly-Asp-Gly-TKPPR;
wherein Bz-MA represents the group benzoylmercaptoacetic acid.

In a preferred embodiment of the invention, the chelators conform to the above formula (II)

In yet another embodiment of the present invention, the peptide is coupled to metal chelators disclosed in co-pending United States application in the name of Pollak et al, filed on 22 July 1994, serial No. 08/279,155 that conform to the formula (III): 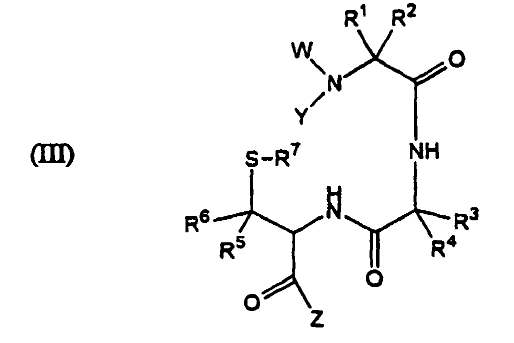 wherein
W is a linear or branched, saturated or unsaturated C₁₋₄alkyl chain that is optionally interrupted by one or two heteroatoms selected from N, O and S; and is optionally substituted by at least one group selected from halogen, hydroxyl, amino, carboxyl, C₁₋₄alkyl, aryl and C(O)Z;
Y is H or a substituent defined by W;
W and Y may together form a 5- to 8-membered saturated or unsaturated heterocyclic ring optionally substituted by at least one group selected from halogen, hydroxyl, amino, carboxyl, oxo, C₁₋₄alkyl, aryl and C(O)Z;
R¹ through R⁴ are selected independently from H; carboxyl; C₁₋₄alkyl; C₁₋₄alkyl substituted with a group selected from hydroxyl, amino, sulfhydryl, halogen, carboxyl, C₁₋₄alkoxycarbonyl and aminocarbonyl; an alpha carbon side chain of a D- or L-amino acid other than proline; and C(O)Z;
R⁵ and R⁶ are selected independently from H; carboxyl; amino; C₁₋₄alkyl; C₁₋₄alkyl substituted by hydroxyl, carboxyl or amino; and C(O)Z;
R⁷ is selected from H and a sulfur protecting group; and
Z is selected from a targeting molecule comprising the sequence Thr-X-Pro-Pro-Arg wherein X is as previously defined.

In a preferred embodiment of the invention, R¹-R⁷, W, X, Y and Z of the above formula (III) are selected to give the chelator DMG-Ser-Cys(Acm) wherein DMG represents the amino acid residue analogue N',N-dimethylglycine. Conjugates of the peptide Thr-X-Pro-Pro-Arg incorporating this chelator include:
DMG-Ser-Cys(Acm)-G-TKPPR;
DMG-Ser-Cys(Adm)-G-TOPPR;
DMG-Ser-Cys(Acm)-βAla-TKPPR; and
DMG-Ser-Cys(Acm)-βAla-βAla-TKPPR.

In another particular embodiment, conjugates of the peptide Thr-X-Pro-Pro-Arg have the chelator coupled to the C-terminus of the peptide. Conjugates of this type include: 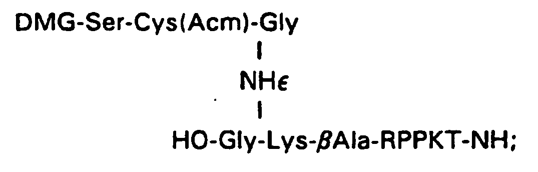 and

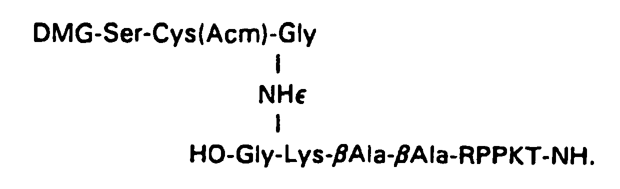

Conjugates of the invention may be prepared by various methods depending upon the chelator chosen. The peptide portion of the conjugate is most conveniently prepared by techniques generally established in the art of peptide synthesis, such as the solid-phase approach. Solid-phase synthesis involves the stepwise addition of amino acid residues to a growing peptide chain that is linked to an insoluble support or matrix, such as polystyrene. The C-terminus residue of the peptide is first anchored to a commercially available support with its amino group protected with an N-protecting agent such as a t-butyloxycarbonyl group (tBoc) or a fluorenylmethoxycarbonyl (FMOC) group. The amino protecting group is removed with suitable deprotecting agents such as TFA in the case of tBOC or piperidine for FMOC and the next amino acid residue (in N-protected form) is added with a coupling agent such as dicyclocarbodiimide (DCC). Upon formation of a peptide bond, the reagents are washed from the support. After addition of the final residue, the peptide is cleaved from the support with a suitable reagent such as trifluoroacetic acid (TFA) or hydrogen fluoride (HF).

Peptide and chelator components are coupled to form a conjugate by reacting the free amino group of the Thr residue of the peptide with an appropriate functional group of the chelator such as a carboxyl group or activated ester. For example, a conjugate may incorporate the chelator ethylenediaminetetraacetic acid (EDTA), common in the art of coordination chemistry, when functionalized with a carboxyl substituent on the ethylene chain. Synthesis of EDTA derivatives of this type are reported in Arya et al, (Bioconjugate Chemistry 1991, 2:323) wherein the four coordinating carboxyl groups are each blocked with a t-butyl group while the carboxyl substituent on the ethylene chain is free to react with the amino group of the peptide thereby forming a conjugate.

A conjugate may incorporate a metal chelator component that is peptidic and compatible with solid-phase peptide synthesis. In this case the chelator may be coupled to the peptide in the same manner as EDTA described above or more conveniently the chelator and peptide are synthesized *in toto* starting from the C-terminal residue of the peptide and ending with the N-terminal residue of the chelator.

Conjugates may further incorporate a linking group component that serves to couple the peptide to the chelator while not adversely affecting either the targetting function of the peptide or the metal binding function of the chelator. Suitable linking groups include amino acid chains and alkyl chains functionalized with reactive groups for coupling to both the peptide and the chelator. An amino acid chain is the preferred linking group when a chelator is peptidic so that the conjugate can be synthesized *in toto* by solid-phase techniques. In a particular embodiment, linking groups are amino acid chains of 1-5 residues and more particularly 1-3 residues. Preferred linking groups include -Gly- and -Gly-Asp-Gly- as well as chains of synthetic amino acid residues -βAla- and -βAla-βAla-.

An alkyl chain linking group may be incorporated in the conjugate by reacting the amino group of the Thr residue of the peptide with a first functional group on the alkyl chain such as a carboxyl group or an activated ester. Subsequently the chelator is attached to the alkyl chain to complete the formation of the conjugate by reacting a second functional group on the alkyl chain with an appropriate group on the chelator. The second functional group on the alkyl chain is selected from substituents that are reactive with a functional group on the chelator and preferably not reactive with the Thr residue of the peptide. For example, when the chelator incorporates a functional group such as a carboxyl group or an activated ester, the second functional group of the alkyl chain linking group can be an amino group. It will be appreciated that formation of the conjugate may require protection and deprotection of the functional groups present in order to avoid formation of undesired products. Protection and deprotection is accomplished using protecting groups, reagents and protocols common in the art of organic synthesis. Particularly, protection and deprotection techniques employed in solid phase peptide synthesis described above may be used.

An alternative chemical linking group to an alkyl chain is polyethylene glycol (PEG) which is functionalized in the same manner as the alkyl chain described above for incorporation in the conjugates. It will be appreciated that linking groups may alternatively be coupled first to the chelator and then to the peptide.

In accordance with one aspect of the invention, peptide-chelator conjugates incorporate a diagnostically useful metal capable of forming a complex. Suitable metals include radionuclides such as technetium and rhenium in their various forms such as^{99m}TcO³⁺, ^{99m}TcO₂ ⁺, ReO³⁺ and ReO₂⁺. Incorporation of the metal within the conjugate can be achieved by various methods common in the art of coordination chemistry. When the metal is technetium-99m, the following general procedure may be used to form a technetium complex. A peptide-chelator conjugate solution is formed initially by dissolving the conjugate in aqueous alcohol such as ethanol. The solution is then degassed to remove oxygen then thiol protecting groups are removed with a suitable reagent, for example with sodium hydroxide and then neutralized with an organic acid such as acetic acid (pH 6.0-6.5). In the labelling step, sodium pertechnetate obtained from a molybdenum generator is added to a solution of the conjugate with an amount of a reducing agent such as stannous chloride sufficient to reduce technetium and heated. The labelled conjugate may be separated from contaminants ^{99m}TcO₄⁻ and colloidal ^{99m}TcO₂ chromatographically, for example with a C-18 Sep Pak cartridge.

In an alternative method, labelling can be accomplished by a transchelation reaction. The technetium source is a solution of technetium complexed with labile ligands facilitating ligand exchange with the selected chelator. Suitable ligands for transchelation include tartarate, citrate and heptagluconate. In this instance the preferred reducing reagent is sodium dithionite. It will be appreciated that the conjugate may be labelled using the techniques described above, or alternatively the chelator itself may be labelled and subsequently coupled to the peptide to form the conjugate; a process referred to as the "prelabelled ligand" method.

Another approach for labelling conjugates of the present invention involves techniques described in a co-pending United States application 08/152,680 filed 16 November 1993. Briefly, the peptide-chelator conjugates are immobilized on a solid-phase support through a linkage that is cleaved upon metal chelation. This is achieved when the chelator is coupled to a functional group of the support by one of the complexing atoms. Preferably, a complexing sulfur atom is coupled to the support which is functionalized with a sulfur protecting group such as maleimide.

When labelled with a diagnostically useful metal, peptide-chelator conjugates of the present invention can be used to detect sites of inflammation by procedures established in the art of diagnostic imaging. A conjugate labelled with a radionuclide metal such as technetium-99m may be administered to a mammal by intravenous injection in a pharmaceutically acceptable solution such as isotonic saline. The amount of labelled conjugate appropriate for administration is dependent upon the distribution profile of the chosen conjugate in the sense that a rapidly cleared conjugate may be administered in higher doses than one that clears less rapidly. Unit doses acceptable for imaging inflammation are in the range of about 5-40 mCi for a 70kg individual. In vivo distribution and localization is tracked by standard scintigraphic techniques at an appropriate time subsequent to administration; typically between 30 minutes and 180 minutes depending upon the rate of accumulation at the target site with respect to the rate of clearance at non-target tissue.

The following examples are presented to illustrate certain embodiments of the present invention.

### Example 1 Preparation of Conjugates:

Pic-Ser-Cys(Acm)-G-TKPPR
Pic-Ser-Cys (Acm)-GDG-TKPPR
Bz-MA-Ser-Cys-GDG-TKPPR

Peptide-chelator conjugates were synthesized *in toto* using 9-fluorenylmethyloxycarbonyl (FMOC) chemistry on an 2-methoxy-4-alkoxybenzyl alcohol resin preloaded with the protected C-terminus residue (Sasrin resin, Bachem Biosciences Inc., Philadelphia PA) using an Applied Biosystems 433A peptide synthesizer (Foster City, CA). N-terminus residues Pic and Bz-MA were incorporated by using picolinic acid and benzoylmercaptoacetic acid respectively as the final residue in the synthesis.

The resin bound conjugate was dried in vacuo for 12 hours. Cleavage of the conjugate from the resin involved mixing a cooled solution of 95% trifluoroacetic acid (TFA) and 5% water (1mL per 100mg of peptide-resin) with the conjugate-resin for 1.5 to 2 hours at room temperature. The resin was removed by filtration and washed 3 times with 30mL t-butyl methyl ether in a 50mL conical polypropylene centrifuge tube forming a white precipitate. The precipitate was dissolved in water with added acetonitrile. The precipitate was frozen in acetone-dry ice and lyophilized over 12 hours. The resulting white powder was dissolved in water, filtered through a 0.45µm syringe filter (Gelman Acrodisc LC PVDF) and purified by reversed-phase HPLC (Beckman System Gold) with a C₁₈ column (Waters RCM 25 x 10) using 0.1 % TFA in water as buffer A and 0.1 % TFA in acetonitrile as buffer B. The column was equilibrated with 100:0 buffer A:buffer B and eluted with a linear gradient in 25 min at 1 mL/min to 50% buffer B.

Fractions were reanalysed on the HPLC and pooled according to matching profiles. When necessary the pooled fractions were repurified using the same conditions. The pure fractions were frozen in acetone-dry ice and lyophilized over 10 hours to give a white powder.

In a like manner, the conjugates DMG-Ser-Cys(Acm)-G-TKPPR; DMG-Ser-Cys(Acm)-G-TQPPR; DMG-Ser-Cys(Acm)-βAla-TKPPR; and DMG-Ser-Cys(Acm)-βAla-βAla-TKPPR were synthesized using the commercially available residue N',N-dimethyl-glycine (DMG) as the final residue in the synthesis. 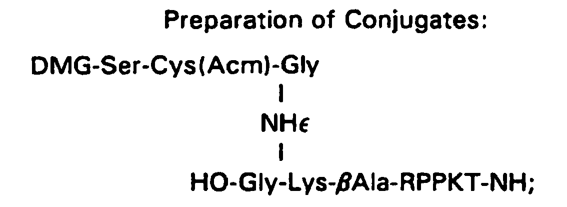 and

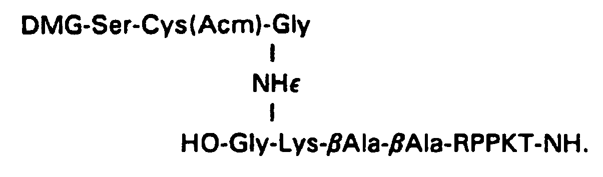

The peptide Thr-X-Pro-Pro-Arg can be coupled at its C-terminus to a chelator or linking group attached thereto that comprise lysine residues via the ε-amino group of the lysine residue by the following procedure. For the titled conjugates, the peptide and part of the linker (TKPPR-βAla-Lys-Gly-OH and TKPPR-βAla-βBAla-Lys-Gly-OH) were initially synthesized using 9-fluorenylmethyloxycarbonyl (FMOC) chemistry with an FMOC-glycine preloaded 2-methoxyl-4-alkoxyl-benzyl alcohol resin and a 1-(4,4-dimethyl-2,6-dioxocyclohexylidine)-ethyl (Dde) orthogonal protected lysine with an Applied Biosystems 433A peptide synthesizer. The peptide-resin was removed from the synthesizer and dried 2 hours in vacuo.

Peptide-resin (50 mg/2ml) was swirled with a 2% hydrazine hydrate in N-methylpyrrolidone (NMP) solution for 3 minutes two times then filtered and washed with DCM and dried in vacuo 4 hours to remove the ε-amino lysine protecting group (Dde).

The chelator portion DMG-Ser-Cys(Acm)-Gly-OH was added to the ε-amino lysine of the peptide on the 433A peptide synthesizer. The chelator-peptide-resin was dried in vacuo 2 hours. Cleavage from the resin and protecting groups involved mixing a cooled solution of 10mL trifluoroacetic acid (TFA), 0.75g phenol, 0.25mL 1,2-ethanediol, 0.5mL thioanisole and 0.5mL water with the chelator-peptide-resin for 2.5 to 3 hours at room temperature. The resin was removed by filtration and washed with 1-3 ml of TFA to obtain 6-8 ml of a clear yellow liquid. This liquid was slowly dropped into 30-35 ml of tert-butyl methyl ether at 0° C in a 50 ml conical polypropylene centrifuge tube forming a white precipitate. The precipitate was centrifuged at 7000 rpm, 0°C for 10 minutes (Sorvall RT 6000, Dupont), decanted and washed two more times with t-butyl methyl ether. Following drying under vacuum the precipitate was dissolved in water. The precipitate was frozen in acetone-dry ice and lyophilized over 10 hours. The resulting white powder was dissoved in dimethylsulfoxide (20 µL) and 50:50 acetonitrile:water solution (980 µL), filtered through a 0.45 *µ*m syringe filter (Gelman Acrodisc LC PVDF), and purified by reversed-phase HPLC (Beckman System Gold) with a C18 column (Waters RCM 25 X 10) using 0.1 % TFA in water as buffer A and 0.1 % TFA in acetonitrile as buffer B. The column was equilibrated with 50:50 buffer A:buffer B and eluted with a linear gradient in 25 min at 1 ml/min to 100% buffer B. Fractions were reanalysed on the HPLC and pooled according to matching profiles. The pure fractions were frozen in acetone-dry ice and lyophilized over 12 hours to give a white powder.

### Example 2 Labelling and Imaging of Conjugates

Imaging studies were performed in a rat inflammation model as follows. Male Wistar rats (Charles River, 150-200g) were injected intramuscularly with (25mg) zymosan, a yeast cell wall suspension, into their left hindlegs 24 hours before imaging. Focal inflammation in the leg was visually detectable after 1 day.

Each conjugate (50µL, 2mg/mL saline) was placed in a 1.5mL tube with 100µL saline, 100µL pertechnetate (10mCi) and 100µL stannous gluconate (50µg stannous chloride and 1 mg sodium gluconate). The tube was capped and placed in a boiling water bath for 10 minutes and then filtered through a Watman PVDF syringe to collect the labelled conjugate solution which was further diluted with saline to prepare an injectable solution (200 µL) containing about 100 µCi (3.7 MBq) of activity. The rats were anaesthetized with somnotol (40 to 50 mg/kg), and the Tc-99m labelled conjugate solution (200µL) was injected intravenously via the tail vein. Serial whole-body scintigrams were acquired 30 minutes after administration with a gamma camera. The rats were then killed with anaesthesia and samples of organs, urine, blood, inflamed muscle (left leg) and non-inflamed muscle (right leg) were weighed and counted in either a well-type gamma counter or in a gamma dose calibrator. The blood dose calculations were made based on assumptions, (1) that the rat weighed 200g and (2) that the blood volume constituted 8% of body weight. Results presented in the following table are averages of multiple trials and are corrected for the residual dose in the tail.

| Chelator | Linking Group | Peptide | % dose/g | | | | | | |
|---|---|---|---|---|---|---|---|---|---|
| | | | Time (min) | Urine | G.I. tract | Blood | Inflam muscle | Uninfl muscle | Inflam: Uninfl |
| Pic-Ser-Cys(Acm)- | -Gly- | -TKCPPR-OH | 30 | 44.71 | 5.08 | 0.392 | 0.154 | 0.052 | 3.6 |
| | | | 180 | 80.62 | 3.71 | 0.048 | 0.026 | 0.006 | 4.7 |
| | | -TQPPR-OH | 35 | 62.3 | 4.4 | 0.149 | 0.081 | 0.018 | 4.8 |
| | | -TKPR-OH | 35 | 22.8 | 22.8 | 0.268 | 0.120 | 0.040 | 3.0 |
| | | -KPPR-OH | 45 | 43.5 | - | 0.220 | 0.125 | 0.059 | 2.1 |
| | | -TKKPR-OH | 35 | 16.1 | 4.2 | 0.136 | 0.071 | 0.022 | 3.2 |
| | | -RTXPR-OH | 35 | 45.4 | 15.0 | 0.169 | 0.099 | 0.033 | 3.0 |
| | | -PRTKP-OH | 35 | 52.5 | 27.1 | 0.089 | 0.046 | 0.019 | 2.6 |
| DMG-Ser-Cys(Acm)- | -Gly- | -TKPPR-OH | 30 | 46.2 | 3.4 | 0.272 | 0.070 | 0.014 | 5.0 |
| | | -TKPRTKPR | 35 | 52.1 | 15.8 | 0.159 | 0.082 | 0.020 | 4.1 |
| | | -TOPPR-OH | 35 | 43.4 | 7.8 | 0.127 | 0.073 | 0.015 | 4.8 |
| DMG-Ser-Cys(Acm)- | -βAls- | -TKPPR-OH | 60 | 81.1 | 2.1 | 0.062 | 0.035 | 0.007 | 5.1 |
| DMG-Ser-Cys(Acm)- -βAls-βAls- | | -TKPPR-OH | 60 | 82.5 | 1.8 | 0.050 | 0.032 | 0.012 | 4.2 |
| Br-MA-Ser-Cys(Acm)- | -GDG- | -TKPPR-OH | 35 | 47.8 | - | 0.483 | 0.226 | 0.057 | 4.0 |
| Pic-Ser-Cys-(Acm) | -GDG- | -TKPPR-OH | 35 | 63.1 | - | 0.228 | 0.166 | 0.034 | 4.9 |
| DMG-Ser-cys(Acm)- | 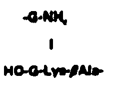 | -RPPKT-NH | 65 | 80.6 | 3.6 | 0.080 | 0.030 | 0.008 | 3.96 |
| DMG-Ser-Cys(Acm)- | 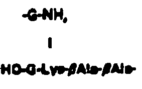 | -RPPKT-NH | 60 | 82.5 | 2.5 | 0.050 | 0.028 | 0.005 | 5.8 |
| Pic-Ser-Cys (Acm)- | 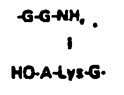 | -RPPKTfor | 35 | 12.2 | 11.3 | 0.748 | 0.213 | 0.086 | 2.5 |
| Pic-Ser-Cys(Acm)- | 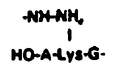 | -RPPKTfor | 35 | 14.3 | 9.2 | 0.485 | 0.163 | 0.062 | 2.6 |

While not wishing to be bound by theory, it is believed that good images are obtained when an agent accumulates selectivity at the target tissue which is indicated by a high target to background. Other factors that dictate the usefulness of an imaging agent are its biodistribution and rate of clearance. For example, an agent that accumulates in the gastrointestinal tract will obscure an image when the target site is in that vicinity as opposed to one that is rapidly excreted through the kidneys into the urine.

Results outlined in the table indicate that conjugates comprising the TKPPR or TQPPR peptide coupled to chelators at its N-terminus, had significantly higher inflamed to uninflamed muscle ratios (target to background) as well as superior distribution profiles compared to the other tuftsin related peptides tested. As a result these conjugates gave images that most clearly differentiated the inflamed muscle tissue. The peptide was coupled to an N₂S₂ and an N₃S class chelator both of which exhibited high target to background and rapid clearance into the urine. Three different linking groups, varying from 1 to 4 amino acids long, were used to couple the peptide to the chelators each of which yielded good images.

The native tuftsin peptide and other antagonists were coupled to the N₃S chelator and tested however each showed target to background ratios lower than the TKPPR and TQPPR conjugates as well as high accumulation in the Gl tract with one exception. The TKKPR conjugate had low accumulation in the Gl tract and blood however the amount in the urine was particularly low which indicates that this conjugate had accumulated elsewhere which would be undesirable for imaging. Coupled to the chelator at its C-terminus, the TKPPR peptide gave relatively low target to background and high Gl tract accumulation resulting in a less resolved image.

The results indicate that conjugates incorporating the peptides TKPPR or TQPPR, coupled to a chelator at its N-terminus, are useful for imaging inflammation and that this indication is independent of the type of chelator or linking group incorporated in the conjugate.

## Claims

1. A peptide-chelator conjugate useful for imaging sites of inflammation, comprising a metal chelator coupled to a peptide comprising the sequence Thr-X-Pro-Pro-Arg, wherein X is an amino acid residue or an analogue thereof, provided that the peptide chelator conjugate is not:
Pic-Ser-Cys-Gly-TKPPR;
Pic-Ser-Cys(Acm)-Gly-TKPPR;
2-Quinolinic acid-Ser-Cys(Acm)-Gly-TKPPR;
1-Quinolinic acid-Ser-Cys(Acm)-Gly-TKPPR;
3-Quinolinic acid-Ser-Cys(Acm)-Gly-TKPPR;
Indole-2-carboxylic acid-Ser-Cys(Acm)-Gly-TKPPR;
Pyrrole-2-carboxylic acid-Ser-Cys(Acm)-Gly-TKPPR;
Ser-Cys-Gly-TKPPR; or
S-Acm-Mercaptoacetyl-Ser-N-methylhydrazino-nicotinic acid-Gly-Thr-Lys-Pro-Pro-Arg

2. A peptide-chelator conjugate according to claim 1, wherein X is an amino acid residue or an analogue thereof having a side chain that incorporates a group that is charged or polar under physiological conditions.

3. A peptide-chelator conjugate according to any preceding claim, wherein the metal chelator and the peptide are coupled by a linking group.

4. A peptide-chelator conjugate according to any preceding claim, wherein the metal chelator is coupled to the N-terminus of the peptide.

5. A peptide-chelator conjugate according to any preceding claim, wherein the metal chelator is coupled to the C-terminus of the peptide.

6. A peptide-chelator conjugate according to any preceding claim, wherein the metal chelator has a general formula: 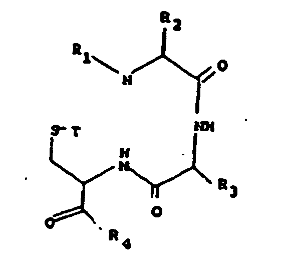
wherein R₁ and R₂ together form a 5- or 6-membered heterocyclic ring which is optionally fused to a 5- or 6-membered ring wherein either ring is optionally substituted with groups selected from alkyl, alkoxy, caroxyl, halogen, hydroxyl and a linking group;
R₃ is selected from H; alkyl, and alkyl substituted by a group selected from amino, aminoacyl, carboxyl, guanidinyl, hydroxyl, thiol, phenyl, phenolyl, indolyl and imidazolyl;
R₄ is selected from hydroxyl, alkoxy, and a linking group and T represents H or a sulfur protecting group.

7. A peptide-chelator conjugate according to claim 6, wherein the peptide is coupled to the metal chelator at R₄.

8. A peptide-chelator conjugate according to claim 6, wherein the peptide is coupled to the metal chelator by a linking group at R₄.

9. A peptide-chelator conjugate according to any one of claims 1 to 8, in a form complexed with a diagnostically useful metal or an oxide or nitride thereof.

10. A peptide-chelator conjugate according to claim 1, selected from:
Pic-Ser-Cys(Acm)-GDG-TKPPR;
Pic-Ser-Cys(Acm)-G-TQPPR;
DMG-Ser-Cys(Acm)-G-TKPPP;
DMG-Ser-Cys(Acm)-G-TQPPR;
DMG-Ser-Cys(Acm)-βAla-TKPPR;
DMG-Ser-Cys(Acm)-βAla-βAla-TKPPR;
Bz-MA-Ser-Cys(Acm)-GDG- TKPPR; 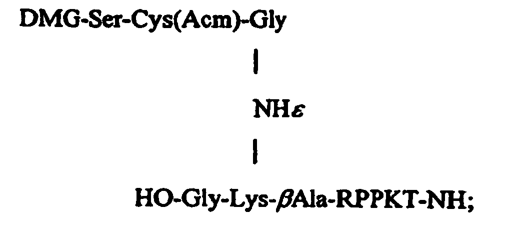 and

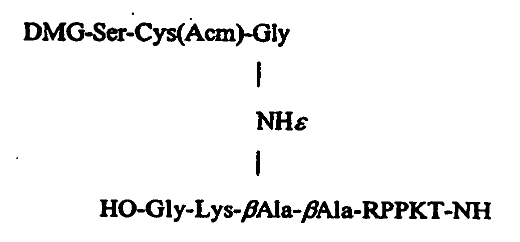

11. The use of a diagnostically effective amount of a composition comprising a peptide-chelator according to any preceding claim in the preparation of an agent for imaging a site of inflammation in a mammal.

## Patentansprüche

1. Peptid-Chelator-Konjugat, das zur Bildgebung von Entzündungsstellen nützlich ist, mit einem Metall-Chelator, der mit einem Peptid gekoppelt ist, welches die Sequenz Thre-X-Pro-Pro-Arg aufweist, wobei X ein Aminosäurerest oder ein Analog davon ist, mit der Maßgabe, dass das Peptid-Chelator-Konjugat nicht
Pic-Ser-Cys-Gly-TKPPR;
Pic-Ser-Cys(Acm)-Gly-TKPPR;
2-Chinolinsäure-Ser-Cys(Acm)-Gly-TKPPR;
1-Chinolinsäure-Ser-Cys (Acm)G-ly-TKPPR;
3-Chinolinsäure-Ser-Cys(Acm)-Gly-TKPPR;
Indol-2-Carbonsäure-Ser-Cys(Acm)-Gly-TKPPR;
Pyrrol-2-Carbonsäure-Ser-Cys(Acm)-Gly-TKPPR;
Ser-Cys-Gly-TKPPR; oder
S-Acm-Mercaptoacetyl-Ser-N-Methylhydrazino-Nikontinsäure-Gly-Thr-Lys-Pro-Pro-Arg
ist.

2. Peptid-Chelator-Konjugat nach Anspruch 1, worin X ein Aminosäurerest oder ein Analog davon ist mit einer Seitenkette, die eine Gruppe mit einschließt, die unter physiologischen Bedingungen geladen oder polar ist.

3. Peptid-Chelator-Konjugat nach einem vorhergehenden Anspruch, wobei der Metall-Chelator und das Peptid durch eine Linker-Gruppe gekoppelt sind.

4. Peptid-Chelator-Konjugat nach einem vorhergehenden Anspruch, wobei der Metall-Chelator an den N-Terminus des Peptids gekoppelt ist.

5. Peptid-Chelator-Konjugat nach einem vorhergehenden Anspruch, wobei der Metall-Chelator an den C-Terminus des Peptids gekoppelt ist.

6. Peptid-Chelator-Konjugat nach einem vorhergehenden Anspruch, wobei der Metall-Chelator eine allgemeine Formel: 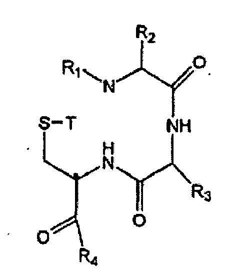 hat, worin R₁ und R₂ zusammen einen 5- oder 6-gliedrigen heterocyclischen Ring bilden, welcher gegebenenfalls mit einem 5- oder 6-gliedrigen Ring verschmolzen ist, worin jeder Ring gegebenenfalls mit Gruppen substituiert ist, die ausgewählt sind aus Alkyl, Alkoxy, Carboxyl, Halogen, Hydroxyl und einer Linker-Gruppe;
R₃ ausgewählt ist aus H; Alkyl und Alkyl, das substituiert ist durch eine Gruppe ausgewählt aus Amino, Aminoacyl, Carboxyl, Guanidinyl, Hydroxyl, Thiol, Phenyl, Phenolyl, Indolyl und Imidazolyl;
R₄ ausgewählt ist aus Hydroxyl, Alkoxy und einer Linker-Gruppe, und
T H oder eine Schwefel-Schutzgruppe darstellt.

7. Peptid-Chelator-Konjugat nach Anspruch 6, wobei das Peptid bei R₄ mit dem Metall-Chelator gekoppelt ist.

8. Peptid-Chelator-Konjugat nach Anspruch 6, wobei das Peptiddurch eine Linker-Gruppe bei R₄ mit-dem-Metall-Chelator gekoppelt ist.

9. Peptid-Chelator-Konjugat nach einem der Ansprüche 1 bis 8 in einer Form, die mit einem diagnostisch nützlichen Metall oder einem Oxid oder Nitrid davon komplexiert ist.

10. Peptid-Chelator-Konjugat nach Anspruch 1, ausgewählt aus
Pic-Ser-Cys(Acm)-GDG-TKPPR;
Pic-Ser-Cys(Acm)-G-TQPPR;
DMG-Ser-Cys(Acm)-G-TKPPR;
DMG-Ser-Cys(Acm)-G-TQPPR;
DMG-Ser-Cys(Acm)-βAla-TKPPR;
DMG-Ser-Cys(Acm)-βAla-βAla-TKPPR;
Bz-Ma-Ser-Cys(Acm)-GDG-TKPPR;
DMG-Ser-Cys(Acm)-Gly
NHε
HO-Gly-Lys-βAla-RPPKT-NH;
und
DMG-Ser-Cys(Acm)-Gly
NHε
HO-Gly-Lys-βAla-βAla-RPPKT-NH.

11. Verwendung einer diagnostisch wirksamen Menge einer Zusammensetzung, die einen Peptid-Chelator gemäß einem vorhergehenden Anspruch umfasst, bei der Herstellung eines Mittels zur Bildgebung einer Entzündungsstelle bei einem Säuger.

## Revendications

1. Conjugué peptide-chélateur utile pour visualiser des sites d'inflammation, comprenant un chélateur métallique couplé à un peptide comprenant la séquence Thr-X-Pro-Pro-Arg, où X est un résidu d'acide aminé ou un analogue de celui-ci, à condition que le conjugué de peptide-chélateur ne soit pas :
Pic-Ser-Cys-Gly-TKPPR ;
Pic-Ser-Cys(Acm)-Gly-TKPPR :
Acide 2-quinolinique-Ser-Cys(Acm)-Gly-TKPPR ;
Acide 1-quinolinique-Ser-Cys (Acm)-Gly-TKPPR ;
Acide 3-quinolinique-Ser-Cys (Acm)-Gly-TKPPR ;
Acide indole-2-carboxylique-Ser-Cys(Acm)-Gly-TKPPR ;
Acide pyrrole-2-carboxylique-Ser-Cys(Acm)-Gly-TKPPR ;
Ser-Cys-Gly-TKPPR ;
S-Acm-mercaptoacétyl-Ser-acide N-méthylhydrazinonicotinique-Gly-Thr-Lys-Pro-Pro-Arg.

2. Conjugué peptide-chélateur selon la revendication 1, dans lequel X est un résidu d'acide aminé ou un analogue de celui-ci ayant une chaîne latérale qui incorpore un groupement qui est chargé ou polaire sous des conditions physiologiques.

3. Conjugué peptide-chélateur selon l'une quelconque des revendications précédentes, dans lequel le chélateur métallique et le peptide sont couplés par un groupement de liaison.

4. Conjugué peptide-chélateur selon l'une quelconque des revendications précédentes, dans lequel le chélateur métallique est couplé à l'extrémité N-terminale du peptide.

5. Conjugué peptide-chélateur selon l'une quelconque des revendications précédentes, dans lequel le chélateur métallique est couplé à l'extrémité C-terminale du peptide.

6. Conjugué peptide-chélateur selon l'une quelconque des revendications précédentes, dans lequel le chélateur métallique a une formule générale : 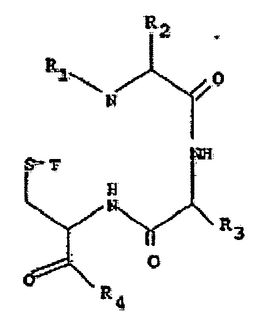 dans laquelle R₁ et R₂ forment conjointement un hétérocycle à 5 ou 6 chaînons qui est facultativement fusionné à un cycle à 5 ou 6 chaînons dans lequel l'un ou l'autre des cycles est facultativement substitué par des groupements choisis parmi les groupements alkyle, alkoxy, carboxyle, halogène, hydroxyle et un groupement de liaison ;
R₃ est choisi parmi H ; alkyle, et alkyle substitué par un groupement choisi parmi les groupements amino, aminoacyle, carboxyle, guanidinyle, hydroxyle, thiol, phényle, phénolyle, indolyle et imidazolyle ;
R₄ est choisi parmi les groupements hydroxyle, alkoxy et un groupement de liaison, et T représente H ou un groupement protecteur du soufre.

7. Conjugué peptide-chélateur selon la revendication 6, dans lequel le peptide est couplé au chélateur métallique à R₄.

8. Conjugué peptide-chélateur selon la revendication 6, dans lequel le peptide est couplé au chélateur métallique par un groupement de liaison à R₄.

9. Conjugué peptide-chélateur selon l'une quelconque des revendications 1 à 8, sous une forme complexée avec un métal utile au point de vue diagnostique ou un oxyde ou nitrure de celui-ci.

10. Conjugué peptide-chélateur selon la revendication 1, choisi parmi :
Pic-Ser-Cys(Acm)-GDG-TKPPR ;
Pic-Ser-Cys(Acm)-G-TQPPR ;
DMG-Ser-Cys(Acm)-G-TKPPR ;
DMG-Ser-Cys (Acm)-G-TQPPR ;
DMG-Ser-Cys (Acm)-βAla-TKPPR ;
DMG-Ser-Cys (Acm) -βAla-βAla-TKPPR ;
Bz-MA-Ser-Cys (Acm)-GDG-TKPPR ; 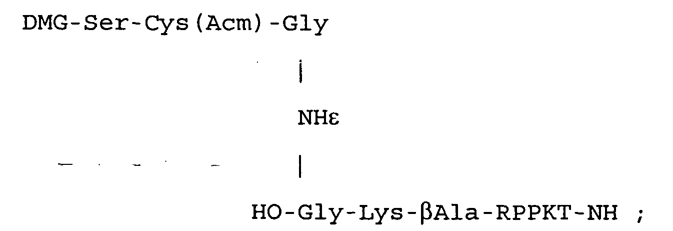 et

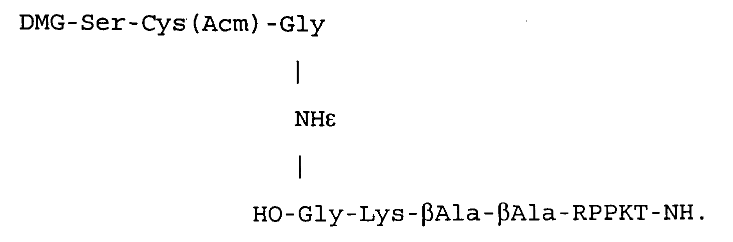

11. Utilisation d'une quantité efficace au point de vue diagnostique d'une composition comprenant un peptide-chélateur selon l'une quelconque des revendications précédentes dans la préparation d'un agent pour visualiser un site d'inflammation chez un mammifère.
